(19) Europäisches Patentamt / European Patent Office / Office européen des brevets

(11) **EP 3 395 443 A1**

(12) **EUROPEAN PATENT APPLICATION**
published in accordance with Art. 153(4) EPC

(43) Date of publication:
**31.10.2018 Bulletin 2018/44**

(21) Application number: **16878922.0**

(22) Date of filing: **22.12.2016**

(51) Int Cl.:
*B01J 38/20* (2006.01)    *B01J 23/887* (2006.01)
*B01J 23/94* (2006.01)    *B01J 38/02* (2006.01)
*B01J 38/12* (2006.01)    *B01J 38/16* (2006.01)
*C07B 61/00* (2006.01)    *C07C 5/48* (2006.01)
*C07C 11/167* (2006.01)

(86) International application number:
**PCT/JP2016/088406**

(87) International publication number:
**WO 2017/111035 (29.06.2017 Gazette 2017/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.12.2015 JP 2015253054**

(71) Applicant: **Nippon Kayaku Kabushiki Kaisha Tokyo 100-0005 (JP)**

(72) Inventors:
• **NAKAZAWA Yuta**
  **Sanyoonoda-shi**
  **Yamaguchi 757-8686 (JP)**
• **OKUMURA Shigeki**
  **Sanyoonoda-shi**
  **Yamaguchi 757-8686 (JP)**

(74) Representative: **Gille Hrabal**
  **Brucknerstrasse 20**
  **40593 Düsseldorf (DE)**

(54) **METHOD FOR REGENERATING CATALYST FOR BUTADIENE PRODUCTION**

(57)    An object of the present invention is to provide a method for regenerating a catalyst for butadiene production, for removing a coke-like substance which is generated by oxidative dehydrogenation of n-butene in the presence of a catalyst for butadiene production and which is attached to the catalyst and the inside of a reactor. After the catalyst is used in oxidative dehydrogenation of butenes, the catalyst regeneration method of the present invention removes a coke-like substance in a reactor which is charged with the catalyst for butadiene production, the catalyst having a prescribed composition before being used in the oxidative dehydrogenation. The catalyst regeneration method includes: a gas treatment step of subjecting the reactor to gas treatment using first gas which contains oxygen at a concentration over 0 vol% to not greater than 21 vol%; and a subsequent gas supply step of supplying, to the reactor, second gas which contains water vapor at a concentration over 0 vol% to not greater than 42 vol% and oxygen at a concentration over 0 vol% to not greater than 21 vol%. The temperature of a heat medium circulating in the reactor is in a range from not less than 200°C to below 400°C, and the temperature of the heat medium circulating in the reactor is fixed from an end of the gas treatment step until an end of the gas supply step. Water vapor contents are different in the first gas and in the second gas.

**Description**

[Technical Field]

The present invention relates to a method for regenerating a catalyst for butadiene production, for removing a coke-like substance which is generated by oxidative dehydrogenation of butenes in the presence of a catalyst for butadiene production and which is attached to the catalyst and the inside of a reactor, and for preventing catalyst damage.

[Background Art]

Butadiene is a raw material for synthetic rubbers and the like, and is conventionally produced on an industrial basis by thermal cracking and extraction of a naphtha fraction. However, since stable supply to the market may be at risk in the future, a new butadiene production method has been desired. As such, a method which attracts attention is oxidative dehydrogenation of butenes, in the presence of a catalyst, from mixed gas which contains butenes and molecular oxygen. According to this method, however, a coke-like substance from a reaction product and/or a reaction by-product is deposited on or attached to the inside of a reactor (namely, the surface and the inside of the catalyst, inert substances, an inner wall of a reaction tube) or the inside of a facility for a follow-up process. The deposited or attached coke-like substance causes various troubles in industrial plants, such as obstruction of reaction gas circulation, clogging of the reaction tube, and plant shut-down or a reduced yield due to such obstruction or clogging.

In order to avoid such troubles, it is a general practice in industrial plants to perform a regeneration treatment for removing a coke-like substance by stopping the reaction before the coke-like substance clogs the reaction tube and then heating the heat medium circulating in the reactor, or in other like manners. Mechanisms of generation of a coke-like substance are assumed, for example, as below. For one, if the catalyst is a composite metal oxide catalyst containing molybdenum, a molybdenum compound precipitates in the reactor by sublimation. Starting from this molybdenum compound, a coke-like substance is formed by polymerization of an olefin and condensation of a high-boiling-point compound. For another, a coke-like substance is formed by polymerization of an olefin and condensation of a high-boiling-point compound, starting from an abnormal acid-base point or a radical formation point in the catalyst or the reactor. For still another, a coke-like substance is formed by generation of a high-boiling-point compound by a Diels-Alder reaction between a conjugated diene and an olefin compound, and condensation of the high-boiling-point compound in a locally low-temperature part of the reactor. There are many other known mechanisms of generation of a coke-like substance.

Methods for removing a coke-like substance deposited on the surface of the catalyst are disclosed in prior art documents. PTL 1 focuses on the fact that the catalyst performance deteriorates due to deposition of a carbon content on a heteropoly acid-based catalyst for producing isobutyric acid or a lower ester thereof. From this point of view, PTL 1 provides a catalyst regeneration method for removing the carbon content from the catalyst by feeding gas which contains air and water vapor into the reactor. In PTL 2, oxygen-containing gas is fed in the reactor in a condition where the peak temperature range of the catalyst layer is between 400°C (equal to the reaction temperature) and 450°C. For removal of a coke-like substance, PTL 2 also discloses it is preferable not to use water vapor together with the oxygen-containing gas. PTL 3 requires, as an essential step for removal of a carbonic substance in a chemical reactor, an oxidation step at a temperature between 400°C and 500°C. PTL 4 discloses a method for regenerating a dehydrogenation catalyst which requires, as an essential step, a step of changing the pressure, repeatedly, rapidly and in opposite directions, by a factor from 2 to 20 within a range from 0.5 bar to 20 bar. PTL 5 discloses a method for regenerating a catalyst for producing a lower aliphatic carboxylic acid ester by allowing a lower olefin and a lower aliphatic carboxylic acid to react in a gas phase. PTL 6 discloses a method for regenerating a zeolite-containing catalyst for converting methanol into dimethyl ether.

PTL 7 discloses a method for oxidative dehydrogenation of butenes ("n-butenes" in PTL 7) to butadiene, characterized by including two or more production steps and at least one regeneration step. The regeneration step is conducted before the conversion loss in a preceding production step exceeds 25% at a constant temperature, by introducing and circulating an oxygen-containing regeneration gas mixture at a temperature between 200 and 450°C. In each regeneration step, 2 to 50 mass% of carbon is burnt up.

Further, PTL 8 discloses a method for oxidative dehydrogenation of butenes ("n-butenes" in PTL 8), including two or more production steps and at least one regeneration step to be conducted between the production steps. The at least two production steps are conducted at a temperature of at least 350°C, and the at least one regeneration step is conducted at a temperature that is at most 50°C above the temperature at which the preceding production step was conducted.

Nevertheless, the burnup of carbon in the regeneration step is a combustion reaction that is difficult to control. Neither PTL 7 nor PTL 8 provides any technique for preventing catalyst damage due to a rapid combustion reaction.

PTL 9 provides a method for oxidative dehydrogenation of butenes ("n-butene" or "n-butenes" in PTL 9) to butadiene in a fixed-bed reactor, with minimum catalyst damage during operation of the fixed-bed reactor. As a process for conducting a regeneration step before excessive carbon deposition occurs in a production step, the oxygen content in the produced gas is adjusted to at least 5% by volume. Besides, PTL 9 puts an emphasis on the timing of the regeneration step, and starts the regeneration step after less than 1000 hours of the production process.

Nevertheless, PTL 9 does not mention any technique for preventing catalyst damage due to the combustion reaction of carbon in the regeneration step.

Actually, the method for removing a coke-like substance needs individual characteristics, depending on the properties of a coke-like substance generated by various reactions as well as the properties of a catalyst. Besides, regarding the production of butadiene by oxidative dehydrogenation of butenes, PTL 1 to PTL 9 do not provide a satisfactory catalyst regeneration method for removing a coke-like substance attached to the catalyst and the inside of the reactor and for preventing catalyst damage. From these points of view, the method for removing a coke-like substance requires further improvement.

[Citation List]

[Patent Literature]

[PTL 1] JP 02-222726 A
[PTL 2] JP 05-192590 A
[PTL 3] JP 2005-521021 A
[PTL 4] JP 2004-522563 A
[PTL 5] JP 2003-71299 A
[PTL 6] JP 58-30340 A
[PTL 7] JP 2016-502549 A
[PTL 8] JP 2016-500372 A
[PTL 9] JP 2016-526565 A

[Summary of Invention]

[Technical Problem]

An object of the present invention is to provide a method for regenerating a catalyst for butadiene production, for removing a coke-like substance which is generated by oxidative dehydrogenation of butenes in the presence of a catalyst for butadiene production and which is attached to the catalyst and the inside of a reactor.

[Solution to Problem]

The present inventors have made intensive researches for solving the above problem and found a catalyst regeneration method which can remove a coke-like substance at a lower temperature than the conventional techniques and which can further prevent catalyst damage, and thereby made the present invention. In order to remove a coke-like substance which is attached to a catalyst for butadiene production and the inside of a reactor due to oxidative dehydrogenation of butenes in the presence of a catalyst for butadiene production having a prescribed composition and charged in the reactor, this catalyst regeneration method includes a step in which the reactor charged with the catalyst used in the oxidative dehydrogenation is subjected to gas treatment using first gas which contains oxygen in a particular proportion, and subsequently, a step in which second gas which contains water vapor and oxygen in particular proportions is supplied to the reactor, wherein a temperature of a heat medium circulating in the reactor is in a range from not less than 200°C to below 400°C, and the temperature of the heat medium circulating in the reactor is fixed from an end of the gas treatment step until an end of the subsequent gas supply step, and wherein a water vapor content in the first gas and a water vapor content in the second gas are controlled to be different.

Specifically, the present invention relates to:

(1) a method for regenerating a catalyst for butadiene production, conducted after the catalyst for butadiene production is used in oxidative dehydrogenation of butenes, for removing a coke-like substance in a reactor which is charged with the catalyst, the catalyst having a composition represented by following Formula 1 before being used in the oxidative dehydrogenation,

$$Mo_{12}Bi_aFe_bCo_cNi_dX_eY_fZ_gO_h \qquad \text{(Formula 1)}$$

wherein X represents at least one alkali metal element selected from the group consisting of lithium, sodium, potassium, rubidium, and cesium, Y represents at least one alkaline earth metal element selected from the group consisting of magnesium, calcium, strontium, and barium, Z represents at least one element selected from the group consisting of lanthanum, cerium, praseodymium, neodymium, samarium, europium, antimony, tungsten, lead, zinc, cerium, and thallium, a, b, c, d, e, f, and g represent atomic ratios of the elements relative to $Mo_{12}$, satisfying ranges of $0.2{\leq}a{\leq}2.0$, $0.6{<}b{<}3.4$, $5.0{<}c{<}8.0$, $0{<}d{<}3.0$, $0{<}e{<}0.5$, $0{\leq}f{\leq}4.0$, and $0{\leq}g{\leq}2.0$, and h is a number that satisfies oxidation states of the other elements. The method includes a step of subjecting the reactor to gas treatment using first gas which contains oxygen at a concentration over 0 vol% to not greater than 21 vol%, and subsequently, a step of supplying, to the reactor, second gas which contains water vapor at a concentration over 0 vol% to not greater than 42 vol% and oxygen at a concentration over 0 vol% to not greater than 21 vol%, wherein a temperature of a heat medium circulating in the reactor is in a range from not less than 200°C to below 400°C, and the temperature of the heat medium circulating in the reactor is fixed from an end of the gas treatment step until an end of the subsequent gas supply step, and wherein a water vapor content in the first gas and a water vapor content in the second gas are different;

(2) the catalyst regeneration method according to (1) above, wherein the gas supply step is conducted after a generation speed of $CO_2$ and CO discharged from the reactor has reached a maximum generation speed during the gas treatment step conducted under a condition defined above, when the generation speed of $CO_2$ and CO discharged from the reactor decreases to 95% or less of the maximum generation speed;

(3) the catalyst regeneration method according to (2) above, wherein a cycle including the gas treatment step and the subsequent gas supply step is repeated twice or more, and wherein a temperature of the heat medium during the gas treatment step and the gas supply step in a first cycle and a temperature of the heat medium during the gas treatment step and the gas supply step in a second cycle are different;

(4) the catalyst regeneration method according to (3) above, wherein the temperature of the heat medium during the gas treatment step and the gas supply step in the second cycle is higher than the temperature of the heat medium during the gas treatment step and the gas supply step in the first cycle;

(5) the catalyst regeneration method according to any one of (1)-(4) above, wherein the temperature of the heat medium circulating in the reactor is between not less than 200°C and not greater than 350°C; and

(6) the catalyst regeneration method according to any one of (1) to (5) above, wherein the catalyst is a supported catalyst for butadiene production in which the catalyst for butadiene production is supported by a support.

[Advantageous Effects of Invention]

The present invention provides a catalyst regeneration method which can remove a coke-like substance at a lower temperature in a shorter time than the conventional techniques, which can further prevent catalyst damage, and which ensures an excellent long-term stability and economic efficiency. In order to remove a coke-like substance which is attached to a catalyst for butadiene production and the inside of a reactor due to oxidative dehydrogenation, this catalyst regeneration method includes a step in which the reactor charged with the catalyst used in the oxidative dehydrogenation is subjected to gas treatment using first gas which contains oxygen in a particular proportion, and subsequently, a step in which second gas which contains water vapor and oxygen in particular proportions is supplied to the reactor, wherein a temperature of a heat medium circulating in the reactor is in a range from not less than 200°C to below 400°C, and the temperature of the heat medium circulating in the reactor is fixed from an end of the gas treatment step until an end of the subsequent gas supply step, and wherein a water vapor content in the first gas and a water vapor content in the second gas are controlled to be different.

[Description of Embodiments]

Hereinafter, the present invention is described in greater detail. The present invention is a method for removing a coke-like substance which is generated by oxidative dehydrogenation of butenes in the presence of a catalyst for butadiene production and which is attached to the catalyst and the inside of a reactor, the catalyst being represented by a following composition formula (Formula 1),

$$Mo_{12}Bi_aFe_bCo_cNi_dX_eY_fZ_gO_h \qquad \text{(Formula 1)}$$

wherein X represents at least one alkali metal element selected from the group consisting of lithium, sodium, potassium, rubidium, and cesium, Y represents at least one alkaline earth metal element selected from the group consisting of magnesium, calcium, strontium, and barium, Z represents at least one element selected from the group consisting of lanthanum, cerium, praseodymium, neodymium, samarium, europium, antimony, tungsten, lead, zinc, cerium, and thallium, a, b, c, d, e, f, and g represent atomic ratios of the elements relative to $Mo_{12}$, satisfying ranges of $0.2 \leq a \leq 2.0$, $0.6 < b < 3.4$, $5.0 < c < 8.0$, $0 < d < 3.0$, $0 < e < 0.5$, $0 \leq f \leq 4.0$, and $0 \leq g \leq 2.0$, and h is a number that satisfies oxidation states of the other elements.

The method for regenerating a catalyst according to the present invention is a catalyst regeneration method, conducted after the catalyst for butadiene production is used in oxidative dehydrogenation of butenes, for removing a coke-like substance in a reactor which is charged with the catalyst, the catalyst having a composition represented by above Formula 1 before being used in the oxidative dehydrogenation. This method includes a step of subjecting the reactor to gas treatment using first gas which contains oxygen at a concentration over 0 vol% to not greater than 21 vol%, and subsequently, a step of supplying, to the reactor, second gas which contains water vapor at a concentration over 0 vol% to not greater than 42 vol% and oxygen at a concentration over 0 vol% to not greater than 21 vol%. In this method, a temperature of a heat medium circulating in the reactor is in a range from not less than 200°C to below 400°C, and the temperature of the heat medium circulating in the reactor is fixed from an end of the gas treatment step until an end of the subsequent gas supply step. A water vapor content in the first gas and a water vapor content in the second gas are different.

In the regeneration method according to the present invention, gas (the first gas and the second gas, respectively) is supplied to the reactor in the gas treatment step and in the gas supply step, under the condition that the temperature of a heat medium circulating in the reactor charged with the catalyst for butadiene production is from not less than 200°C to below 400°C, preferably from not less than 200°C to not greater than 350°C. Further in the regeneration method according to the present invention, the temperature of the heat medium circulating in the reactor is fixed in a range from not less than 200°C to below 400°C, preferably fixed in a range from not less than 200°C to not greater than 350°C, from the end of the gas treatment step until the end of the subsequent gas supply step. As the composition of the first gas employed in the gas treatment step, the oxygen content is from over 0 vol% to not greater than 21 vol%. The water vapor content of the first gas simply needs to be different from that of the second gas, and is preferably less than that of the second gas. More preferably, the first gas is free of water vapor. As the composition of the second gas employed in the gas supply step, the water vapor content is from over 0 vol% to not greater than 42 vol%, and the oxygen content is from over 0 vol% to not greater than 21 vol%.

The "coke-like substance" in the context of the present invention is a substance generated in a reaction for butadiene production, from at least any one of a reaction material, a target product, or a reaction by-product. The chemical composition and generation mechanism of the coke-like substance is not yet clarified in detail. It is known, however, that the coke-like substance is deposited on or attached to the surface and the inside of the catalyst, inert substances, an inner wall of a reaction tube or the inside of a facility for a follow-up process, and thereby causes various troubles particularly in industrial plants, such as obstruction of reaction gas circulation, clogging of the reaction tube, and shut-down of the reaction due to such obstruction or clogging.

Butenes in the context of the present invention mean single-component gases each composed of 1-butene, trans-2-butene, cis-2-butene, or isobutylene, or mixed gases each containing at least one component selected therefrom. Butadiene in the context of the present invention means, more strictly, 1,3-butadiene.

The regeneration method according to the present invention can remove a coke-like substance by supplying gas to the reactor under the condition that the temperature of a heat medium circulating in the reactor is from not less than 200°C to below 400°C, preferably from not less than 200°C to not greater than 350°C. The removal mechanism is not yet clarified in detail, but the temperature of the heat medium in the above-defined ranges seems suitable for gradual decomposition of a coke-like substance without causing drastic combustion of the coke-like substance when the coke-like substance is generated by the oxidative dehydrogenation and attached to the catalyst, the inner wall of a reaction tube, inert substances or the like. On the other hand, under the condition that the temperature of the heat medium is 400°C or greater, a coke-like substance is likely to burn drastically. If the coke-like substance is attached to the catalyst, the heat of combustion may cause a change in the crystal structure of the catalyst and eventual alteration and deterioration of the catalyst, and the pressure of generated combustion gas may even damage the catalyst. Further, if drastic heat generation occurs in the reaction tube, the reactor may be broken. Under the condition that the temperature of the heat medium is less than 200°C, combustion does not proceed as planned, so that the regeneration process may fail to exhibit its advantageous effect sufficiently or may take a longer time. As a result, the plant shut-down period may be extended so much as to lose economic efficiency.

In the gas treatment step, water vapor may be added to diluted oxygen gas which is to be supplied into the reactor. As a result, even if the temperature of the heat medium is low, a coke-like substance can be effectively removed from the reactor. Regarding the gas supplied into the reactor, the percentage of water vapor content and/or the percentage of oxygen content is/are not particularly limited as far as the percentage of water vapor content in the second gas is from over 0 vol% to not greater than 42 vol%, the percentages of oxygen content in the first gas and the second gas are from over 0 vol% to not greater than 21 vol%, and the water vapor content in the first gas and the water vapor content in the second gas are different. The regeneration method according to the present invention removes a coke-like substance by supplying the first gas into the reactor in the gas treatment step, in which the first gas contains oxygen at a concentration over 0 vol% to not greater than 21 vol%, and subsequently by supplying the second gas into the reactor in the gas supply step, in which the second gas contains water vapor at a concentration over 0 vol% to not greater than 42 vol%. Preferably in the regeneration method according to the present invention, the first gas supplied into the reactor in the gas treatment step contains oxygen at a concentration over 0 vol% to not greater than 21 vol% and is free of water vapor, and the second gas supplied into the reactor in the subsequent gas supply step contains water vapor at a concentration over 0 vol% to not greater than 42 vol% and oxygen at a concentration over 0 vol% to not greater than 21 vol%. In this manner, it is possible to remove a coke-like substance more effectively.

The volume percentage of water vapor and/or the volume percentage of oxygen in the gas (the first gas and the second gas) supplied into the reactor may be controlled, for example, by nitrogen or the like.

The timing for switching the composition of the gas supplied into the reactor from the composition for the first gas to the composition for the second gas, namely, the timing for proceeding from the gas treatment step to the gas supply step, is after the generation speed of discharged $CO_2$ and CO has reached the maximum generation speed while the gas treatment step is conducted under the condition defined above, preferably when the generation speed of $CO_2$ and CO discharged from the reactor decreases to 95% or less of the maximum generation speed. A more preferable timing is when the above conditions are met and further when the generation speed has slowed down gently and remains stable. The properties and amount of a coke-like substance are variable depending on the reaction conditions, the reaction scale, the reaction period, and the catalyst performance in the oxidative dehydrogenation, and hence combustion behaviors can very accordingly. Considering such differences, the timing for switching the compositions of the gases to be supplied into the reactor may be suitably adjusted within the above-mentioned range. In this description, the generation speed of discharged $CO_2$ and CO is the generation speed of $CO_2$ and CO generated during the combustion treatment, which is calculated with excluding the amount of $CO_2$ and CO naturally contained in the air.

Normally, the heatup speed for the heat medium during the regeneration treatment is not particularly limited, but is preferably in a range from not less than 1°C/h to not greater than 200°C/h. As the heatup speed for the heat medium during the regeneration treatment, a heatup speed greater than 200°C/h may trigger drastic combustion and may not ensure a sufficient regeneration process, and a heatup speed less than 1°C/h may require a longer time for the regeneration treatment and may sacrifice economic efficiency.

More preferably, the adjustment of the heatup speed is combined with the above-mentioned adjustment of the temperature of the heat medium circulating in the reactor and the above-mentioned adjustment of the volume percentage of water vapor and/or the volume percentage of oxygen in the gases supplied into the reactor. The most preferable mode is to fix the temperature of the heat medium circulating in the reactor at between not less than 200°C and not greater than 350°C, to carry out an operation, twice or more, which includes the regeneration treatment using the gas which contains oxygen at a concentration over 0 vol% to not greater than 21 vol% and the subsequent supply of the gas which contains water vapor at a concentration over 0 vol% to not greater than 42 vol%, until the generation speed of $CO_2$ and CO discharged as the outgoing gas from the reaction tube decreases to a suitable, lowest possible generation speed. In the case where the operation of this process is repeated twice, the temperature of the heat medium in the first operation and the temperature of the heat medium in the second operation are preferably different. It is more preferable if the temperature of the heat medium in the second operation is higher than in the first operation. In the case where the operation of the process is repeated twice or more, the temperature of the heat medium may be changed in every operation. A suitable generation speed is variable depending on the reaction conditions, the reaction scale, the reaction period, and the catalyst performance in the oxidative dehydrogenation, and should be determined appropriately.

In the catalyst regeneration method according to the present invention, a cycle including the gas treatment step and the subsequent gas supply step is repeated twice or more, preferably with the temperature of the heat medium during the gas treatment step and the gas supply step in the first cycle and the temperature of the heat medium during the gas treatment step and the gas supply step in the second cycle being different. More preferably, the temperature of the heat medium during the gas treatment step and the gas supply step in the second cycle is higher than the temperature of the heat medium during the gas treatment step and the gas supply step in the first cycle.

The catalyst employed in the catalyst regeneration method according to the present invention is now described. In some cases, the catalyst regeneration method according to the present invention may present the effect of the present invention also in a method for regenerating a composite metal oxide catalyst which has a known composition containing molybdenum and bismuth as principal components.

The catalyst employed in the present invention is a catalyst for butadiene production which has been used in oxidative dehydrogenation of butenes. The catalyst, before being used in the oxidative dehydrogenation, has a composition (the composition of a catalytically active component) represented by following Formula 1,

$$Mo_{12}Bi_aFe_bCo_cNi_dX_eY_fZ_gO_h \qquad \text{(Formula 1)}$$

wherein X represents at least one alkali metal element selected from the group consisting of lithium, sodium, potassium, rubidium, and cesium, Y represents at least one alkaline earth metal element selected from the group consisting of magnesium, calcium, strontium, and barium, Z represents at least one element selected from the group consisting of lanthanum, cerium, praseodymium, neodymium, samarium, europium, antimony, tungsten, lead, zinc, cerium, and thallium, a, b, c, d, e, f, and g represent atomic ratios of the elements relative to $Mo_{12}$, satisfying ranges of $0.2 \leq a \leq 2.0$, $0.6 < b < 3.4$, $5.0 < c < 8.0$, $0 < d < 3.0$, $0 < e < 0.5$, $0 \leq f \leq 4.0$, and $0 \leq g \leq 2.0$, and h is a number that satisfies oxidation states of the other elements.

A raw material for each of the metal elements for obtaining the catalyst employed in the present invention is not particularly limited, and may be selected from nitrates, nitrites, sulfates, ammonium salts, organic acid salts, acetates, carbonates, subcarbonates, chlorides, inorganic acids, inorganic acid salts, heteropoly acids, heteropoly acid salts, hydroxides, oxides, metals, alloys, etc. or mixtures thereof, each containing at least one of the metal elements, as specifically exemplified below. For molybdenum, a preferable raw material is ammonium molybdate. In particular, ammonium molybdate includes a variety of compounds such as ammonium dimolybdate, ammonium tetramolybdate, ammonium heptamolybdate, etc., of which ammonium heptamolybdate is most preferable. For bismuth, a preferable raw material is bismuth nitrate. For iron, cobalt, nickel, and other elements, standard raw materials are oxides; nitrates, carbonates, organic acid salts, hydroxides or the like which can be oxides on ignition; or mixtures thereof.

The process for preparing the catalyst in the present invention is not particularly limited, but can be roughly grouped into two preparation processes as below. For convenience, the two preparation processes are called Process (A) and Process (B) in the present invention. In Process (A), a catalytically active component is obtained in powdery form, and the catalyst powder is later formed into a desired shape. In Process (B), a preformed support is brought into contact with a solution in which a catalytically active component is dissolved, and is thereby allowed to support the catalyst. Detailed description of Process (A) and Process (B) is given below.

First, a catalyst preparation process by Process (A) is described step by step in order, as a preferable example. However, there is no limitations on the order, number, and combination of the process steps for obtaining a final catalyst product.

<Step (A1): Formulation and drying>

A mixed solution or slurry containing a raw material for a catalytically active component is prepared and subjected to precipitation, gelation, coprecipitation, hydrothermal crystallization, or other like treatment, and then dried by a known drying technique such as drying atomization, evaporation to dryness, drum drying, or freeze-drying, to give dry powder for the present invention. The mixed solution or slurry may contain water, an organic solvent, or a mixed solution thereof as the solvent. The concentration of the raw material for the catalytically active component is not limited, either. Further, the formulation conditions (e.g. liquid temperature, atmosphere) and the drying conditions for the mixed solution or slurry are not particularly limited. Having said that, such conditions should be selected from suitable ranges in consideration of performance, mechanical strength, forming property, production efficiency, and other like factors for the final catalyst. The most preferable process in the present invention is to form a mixed solution or slurry containing a raw material for the catalytically active component at between not less than 20°C and not greater than 90°C, to introduce the mixed solution or slurry into a spray dryer in which the hot-air inlet temperature, the pressure inside the spray dryer, and the slurry flow rate are controlled such that the drier outlet temperature is between not less than 70°C and not greater than 150°C and that the resulting dry powder has a mean particle diameter from not less than 10 μm to not greater than 700 μm.

<Step (A2): Preliminary calcination>

The thus obtained dry powder is subjected to preliminary calcination at from not less than 200°C to not greater than 600°C to give preliminary calcined powder for the present invention. The conditions for the preliminary calcination, such as the time and the atmosphere for the preliminary calcination, are not particularly limited. The preliminary calcination technique is not particularly limited, either, and may be a fluidized bed, a rotary kiln, a muffle furnace, a tunnel calcination furnace or the like. Such conditions should be selected from suitable ranges in consideration of performance, mechanical strength, forming property, production efficiency, and other like factors for the final catalyst. A preferable preliminary calcination in the present invention is performed in a tunnel calcination furnace at from not less than 300°C to not greater than 600°C, for a period from not less than one hour to not greater than 12 hours, in an air atmosphere.

<Step (A3): Forming>

The thus obtained preliminary calcined powder may be directly used as the catalyst in the powdery state, but may be formed into a shape for use. The shape of a formed product is not particularly limited and may be spherical, columnar, or annular. The shape of a formed product should be selected in consideration of mechanical strength, reactor, production efficiency in preparation, and other like factors for the final catalyst obtained through a series of preparation steps. The forming technique is not particularly limited, either. In the case where a support, a forming auxiliary, a strength improving agent, a binder and the like as mentioned later are added to the preliminary calcined powder, a columnar or annular formed product is obtained by means of a tableting press, an extrusion molding machine or the like, and a spherical formed product is obtained by means of a granulator or the like. A preferable supporting/forming technique in the present invention is to coat an inert spherical support with the preliminary calcined powder by tumbling granulation.

A material for the spherical support may be a known material such as alumina, silica, titania, zirconia, niobia, silica alumina, silicon carbide, carbides, and mixtures thereof. In such materials, the particle diameter, water absorption rate, mechanical strength, crystallinity of each crystal phase, mixing proportion, and other like conditions are not particularly limited. Such conditions should be selected from suitable ranges in consideration of performance, forming property, production efficiency or other like factors for the final catalyst. The mixing proportion of the spherical support and the preliminary calcined powder is calculated as a supporting rate based on the weight of the charged raw materials.

$$\text{Supporting rate (wt\%)} = \text{(weight of preliminary calcined powder used in forming)} / \{\text{(weight of preliminary calcined powder used in forming)} + \text{(weight of spherical support used in forming)}\} \times 100$$

Raw materials for the forming, other than the preliminary calcined powder, include a forming auxiliary such as crystalline cellulose, a strength improving agent such as ceramic whisker, a binder such as an alcohol, a diol, a triol, and an aqueous solution thereof, and the like. These materials may be freely selected and blended at an optional mixing proportion with the preliminary calcined powder, and may be used in the forming, without particular limitation. In the case where the binder is a solution containing the above-mentioned raw material for the catalyst, it is possible to introduce an element on the outermost surface of the catalyst in a manner different from Step (A1).

<Step (A4): Main calcination>

The thus obtained preliminary calcined powder or formed product is preferably subjected to re-calcination (main calcination) at from not less than 300°C to not greater than 600°C, prior to use in oxidative dehydrogenation. The conditions for the main calcination, such as the time and the atmosphere for the main calcination, are not particularly limited. The main calcination technique is not particularly limited, either, and may be a fluidized bed, a rotary kiln, a muffle furnace, a tunnel calcination furnace or the like. Such conditions should be selected from suitable ranges in consideration of performance, mechanical strength, production efficiency, and other like factors for the final catalyst. The most preferable main calcination in the present invention is performed in a tunnel calcination furnace at from not less than 450°C to not greater than 600°C, for a period from not less than one hour to not greater than 12 hours, in an air atmosphere. The temperature-rising time is usually in a range from not less than 2 hours to not greater than 20 hours, preferably from not less than 3 hours to not greater than 15 hours, and further preferably from not less than 4 hours to not greater than 10 hours.

Next, a catalyst preparation process by Process (B) is described step by step in order. However, there is no limitations on the order, number, and combination of the process steps for obtaining a final catalyst product.

<Step (B1): Impregnation>

Prepared first is a solution or slurry in which a catalytically active component is introduced. The catalyst obtained in Process (A) or the formed support is impregnated with this solution or slurry to give a formed product. The technique for supporting the catalytically active component by impregnation is not particularly limited and may be dipping, incipient wetness impregnation, ion exchange, pH swing, and the like. The solvent for the solution or slurry may be any of water, an organic solvent, or a mixed solution thereof. The concentration of the raw material for the catalytically active component is not limited. Further for the mixed solution or slurry, the liquid temperature, the pressure to the liquid, and the atmosphere around the liquid are not particularly limited. Such conditions should be selected from suitable ranges in consideration of performance, mechanical strength, forming property, production efficiency, and other like factors for the final catalyst. The shape of the catalyst obtained in Process (A) and the formed support are not particularly limited and may be spherical, columnar, annular, powdery or the like. Additionally, the material quality, particle diameter, water absorption rate, and mechanical strength are not particularly limited.

<Step (B2): Drying>

The thus obtained formed product is subjected to heat treatment in a range from not less than 20°C to not greater than 200°C by a known drying technique, such as evaporation to dryness, drum drying, freeze-drying, and the like, to give a formed dry catalyst product for the present invention. The drying time and the drying atmosphere are not particularly limited. The drying technique is not particularly limited, either, and may be a fluidized bed, a rotary kiln, a muffle furnace, a tunnel calcination furnace or the like. Such conditions should be selected from suitable ranges in consideration of performance, mechanical strength, forming property, production efficiency, and other like factors for the final catalyst.

<Step (B3): Main calcination>

As the main calcination, the thus obtained formed dry catalyst product is subjected to heat treatment at from not less than 300°C to not greater than 600°C, to give a catalyst for the present invention. The conditions for the main calcination, such as the time and the atmosphere for the main calcination, are not particularly limited. The main calcination technique is not particularly limited, either, and may be a fluidized bed, a rotary kiln, a muffle furnace, a tunnel calcination furnace or the like. Such conditions should be selected from suitable ranges in consideration of performance, mechanical strength, formability, production efficiency, and other like factors for the final catalyst. The most preferable main calcination in the present invention is performed in a tunnel calcination furnace at from not less than 450°C to not greater than 600°C, for a period from not less than one hour to not greater than 12 hours, in an air atmosphere. The temperature-rising time is usually in a range from not less than 2 hours to not greater than 20 hours, preferably from not less than 3 hours to not greater than 15 hours, and further preferably from not less than 4 hours to not greater than 10 hours.

The catalyst prepared and obtained in each of the above manners is not particularly limited in terms of shape and size. Having said that, considering the work efficiency in charging the catalyst into the reaction tube, the pressure loss in the reaction tube after the charging, and other such factors, a preferable catalyst has a spherical shape and a mean particle diameter from not less than 3.0 mm to not greater than 10.0 mm, with a supporting rate of the catalytically active component being from not less than 20 wt% to not greater than 80 wt%.

The regeneration method according to the present invention is conducted after oxidative dehydrogenation of n-butene in the presence of a catalyst for butadiene production. The oxidative dehydrogenation is conducted under following reaction conditions. The gas employed in the oxidative dehydrogenation is mixed gas which is composed of n-butene at a concentration from not less than 1 vol% to not greater than 20 vol%, molecular oxygen at a concentration from not less than 5 vol% to not greater than 20 vol%, water vapor at a concentration from not less than 0 vol% to not greater than 60 vol%, inert gas (e.g. nitrogen gas, carbon dioxide gas) at a concentration from not less than 0 vol% to not greater than 94 vol%, as a raw material gas. The temperature of the heat medium is in a range from not less than 200°C to not greater than 500°C. The reaction pressure is from not less than the ordinary pressure to not greater than 10 atm. The gas hourly space velocity (GHSV) of the raw material gas to the catalyst is in a range from not less than 350 $hr^{-1}$ to not greater than 7000 $hr^{-1}$. The reaction mode may be selected, without restriction, from a fixed-bed mode, a moving-bed mode, and a fluidized-bed mode, but is preferably a fixed-bed mode.

The gas hourly space velocity (hereinafter abbreviated as "GHSV") in the catalyst regeneration method according to the present invention is not particularly limited, but is usually in a range from not less than 50 $hr^{-1}$ to not greater than 4000 $hr^{-1}$, and preferably in a range from not less than 100 $hr^{-1}$ to not greater than 2000 $hr^{-1}$. A GHSV greater than the normal range may cause catalyst damage and various problems due to the catalyst damage. Specifically, catalyst activity deteriorates when powder or flakes of the damaged catalyst clog(s) in the reactor or flow(s) out of the reactor. Further, a carbonic substance flowing out to the follow-up process may cause contamination. On the other hand, a GHSV less than the normal range hampers efficient removal of a coke-like substance, so that the regeneration treatment may require a long time or may fail to exhibit its advantageous effect fully.

[Examples]

Hereinafter, the present invention is described in greater detail by way of Examples. However, the present invention should not be limited by these Examples unless going beyond the gist of the invention. In the following description, the unit "%" means "mol%" unless otherwise stated, and the terms "n-butene conversion" and "TOS" are defined as below.

$$\text{n-butene conversion (mol\%)}$$

$$= \text{(molar amount of reacted n-butene/molar amount of}$$

$$\text{supplied n-butene)} \times 100$$

TOS = circulation time of mixed gas (hour)

<Example 1>

(Catalyst preparation)

To start with, 800 parts by weight of ammonium heptamolybdate was dissolved completely in 3000 parts by weight of purified water heated to 80°C (a mother liquor 1). Next, 11 parts by weight of cesium nitrate was dissolved in 124 ml of purified water, and the mixture was added to the mother liquor 1. Then, 275 parts by weight of ferric nitrate, 769 parts by weight of cobalt nitrate, and 110 parts by weight of nickel nitrate were dissolved in 612 ml of purified water heated to 60°C, and the mixture was fed to the mother liquor 1. Further, in an aqueous solution of nitric acid prepared by adding 79 parts by weight of nitric acid (60 wt%) to 330 ml of purified water heated to 60°C, 311 parts by weight of bismuth nitrate was dissolved, and the mixture was fed to the mother liquor 1. The mother liquor 1 was dried by spray drying, and resulting dry powder was subjected to preliminary calcination at 440°C for 5 hours. To the thus obtained preliminary calcined powder, 5 wt% of crystalline cellulose (relative to the preliminary calcined powder) was added and mixed well. Thereafter, using a 33-wt% glycerol solution as a binder, the preliminary calcined powder was formed in a spherical shape by tumbling granulation, so as to be supported by an inert spherical support at a supporting rate of 50 wt%. The spherical formed product was calcined at 520°C for 5 hours to give a supported catalyst for butadiene production, as an example of the catalyst employed in the regeneration method according to the present invention. In this supported catalyst, a catalyst for butadiene production having a composition represented by Formula 1 above, wherein X in Formula 1 was Cs, and f and g in Formula 1 were 0, was supported by a support. The atomic ratio in the catalyst, calculated by the charged raw materials, was Mo:Bi:Fe:Co:Ni:Cs=12:1.7:1.8:7.0:1.0:0.15 (a=1.7, b=1.8, c=7.0, d=1.0, and e=0.15 in Formula 1).

(Deposition reaction of a coke-like substance (oxidative dehydrogenation of butenes))

Using a reactor equipped with a stainless steel reaction tube, 106 ml of the supported catalyst for butadiene production as obtained above was charged in the stainless steel reaction tube. Then, using mixed gas in which the gas volume ratio of 1-butene:oxygen:nitrogen:water vapor was 1:1:7:1, the reaction was conducted under ordinary pressure at a GHSV of 600 hr$^{-1}$, with the temperature of the heat medium circulating in the reactor being changed to keep the 1-butene conversion at 85.0±1.0%. The reaction was continued up to the TOS of 200 hours, thereby allowing a coke-like substance to be deposited on the supported catalyst for butadiene production.

(Removal of the coke-like substance (regeneration treatment))

In order to remove the deposited coke-like substance, a combustion reaction (a gas treatment step in the first cycle) was started under ordinary pressure, with the temperature of the heat medium circulating in the reactor being kept at 250°C, using mixed gas (first gas) in which the gas volume ratio of oxygen:nitrogen:water vapor was 1:9:0 (oxygen content 10 vol%), at a space velocity of the mixed gas of 250 hr$^{-1}$. During the combustion reaction, outgoing gas from the reaction tube was analyzed by a gas chromatograph equipped with a thermal conductivity detector. The total generation speed of $CO_2$ and CO, Rcox, was 0.21 mmol/h.
After Rcox decreased to 0.17 mmol/h (81% of the maximum under the same conditions) (after the end of the gas treatment step in the first cycle), only the gas volume ratio of oxygen:nitrogen:water vapor was changed to 1:7:2 (oxygen content 10 vol%, water vapor content 20 vol%), with the temperature of the heat medium and the space velocity of the mixed gas being fixed at 250°C and 250 hr$^{-1}$, respectively. As a result, Rcox rose to 1.22 mmol/h (a gas supply step in the first cycle).
After Rcox decreased to 0.44 mmol/h (36% of the maximum under the same conditions), the gas volume ratio of oxygen:nitrogen:water vapor was returned to 1:9:0. Thereafter, with Rcox being monitored, the temperature of the heat medium was raised and maintained by 10°C increments at a rate of 200°C/h, repeatedly and gradually up to 280°C (a gas treatment step in the second cycle). When the temperature of the heat medium reached 280°C, Rcox was 0.57 mmol/h.

After Rcox decreased to 0.54 mmol/h (95% of the maximum under the same conditions), nitrogen was substituted with water vapor until the gas composition of oxygen:nitrogen:water vapor became 1:7:2, with the temperature of the heat medium and the space velocity of the mixed gas being fixed at 280°C and 250 hr$^{-1}$, respectively. As a result, Rcox rose to 2.80 mmol/h (a gas supply step in the second cycle).

After Rcox decreased to 1.05 mmol/h (38% of the maximum under the same conditions), the gas volume ratio of oxygen:nitrogen:water vapor was returned to 1:9:0. Thereafter, with Rcox being monitored, the temperature of the heat medium was raised and maintained by 10°C increments at a rate of 200°C/h, repeatedly and gradually up to 350°C. It took 34 hours until the temperature of the heat medium reached 350°C. The catalyst after the regeneration treatment was taken out and visually inspected. The catalyst showed no sign of damage or discoloration.

<Comparative Example 1>

(Catalyst preparation)

A catalyst was prepared in the same manner as in Example 1.

(Deposition reaction of a coke-like substance)

The deposition reaction was conducted in the same manner as in Example 1.

(Removal of the coke-like substance (regeneration treatment))

In order to remove the deposited coke-like substance, a combustion reaction was started under ordinary pressure, with the temperature of the heat medium circulating in the reactor being kept at 240°C, using mixed gas in which the gas volume ratio of oxygen:nitrogen:water vapor was 2:8:0, at a space velocity of 250 hr$^{-1}$. During the combustion reaction, outgoing gas from the reaction tube was analyzed by a gas chromatograph equipped with a thermal conductivity detector. The total generation speed of $CO_2$ and CO, Rcox, was 0.4 mmol/h. After the total generation speed of $CO_2$ and CO decreased and remained stable, with Rcox being monitored, the temperature of the heat medium was raised and maintained by 10°C increments at a rate of 200°C/h, repeatedly and gradually up to 280°C. When the temperature of the heat medium reached 280°C, the total generation speed of $CO_2$ and CO rose to 0.7 mmol/h. Then again, with the total generation speed of $CO_2$ and CO being monitored, the temperature of the heat medium was raised and maintained by 10°C increments at a rate of 200°C/h, repeatedly and gradually up to 340°C. It took 170 hours until the temperature of the heat medium reached 340°C. The catalyst after the regeneration treatment was taken out and visually inspected. The catalyst showed a sign of damage (particularly, disintegration) and a sign of discoloration. The rate of disintegration, obtained by the following formula, was 0.42 wt%:

$$\text{Rate of disintegration (wt\%)} = 100 \times (W_0 - W_1)/W_0$$

wherein $W_0$ is the weight of the catalyst charged in the reaction tube, and $W_1$ is the weight of the catalyst remaining on the sieve when the catalyst was taken out of the reaction tube after the reaction and sieved through a sieve with an aperture width of 3.35 mm.

The following conclusion can be led from the above Examples. In the method for removing a coke-like substance which has been attached to the catalyst or the inside of a reactor due to a reaction for producing butadiene from butenes, the method can be carried out efficiently at a lower temperature, can exhibit the advantageous effect of the present invention, and can prevent catalyst damage, by including the step of supplying mixed gas which contains not only oxygen but also water vapor.

The present invention can be embodied and practiced in other different forms without departing from the spirit and essential characteristics of the present invention. Therefore, the above-described embodiments are considered in all respects as illustrative and not restrictive. The scope of the invention is indicated by the appended claims rather than by the foregoing description. All variations and modifications falling within the equivalency range of the appended claims are intended to be embraced therein.

The present application claims priority to Japanese Patent Application No. 2015-253054, filed December 25, 2015. The contents of all publications, patents, and patent applications (including the Japanese patent application mentioned just above) cited in the present specification are incorporated herein by reference in their entirety.

**Claims**

1.  A method for regenerating a catalyst for butadiene production, conducted after the catalyst for butadiene production is used in oxidative dehydrogenation of butenes, for removing a coke-like substance in a reactor which is charged with the catalyst,
    the catalyst having a composition represented by following Formula 1 before being used in the oxidative dehydrogenation,

$$Mo_{12}Bi_aFe_bCo_cNi_dX_eY_fZ_gO_h \qquad \text{(Formula 1)}$$

    wherein X represents at least one alkali metal element selected from the group consisting of lithium, sodium, potassium, rubidium, and cesium, Y represents at least one alkaline earth metal element selected from the group consisting of magnesium, calcium, strontium, and barium, Z represents at least one element selected from the group consisting of lanthanum, cerium, praseodymium, neodymium, samarium, europium, antimony, tungsten, lead, zinc, cerium, and thallium, a, b, c, d, e, f, and g represent atomic ratios of the elements relative to $Mo_{12}$, satisfying ranges of $0.2{\leq}a{\leq}2.0$, $0.6{<}b{<}3.4$, $5.0{<}c{<}8.0$, $0{<}d{<}3.0$, $0{<}e{<}0.5$, $0{\leq}f{\leq}4.0$, and $0{\leq}g{\leq}2.0$, and h is a number that satisfies oxidation states of the other elements,
    wherein the method comprises:

    a step of subjecting the reactor to gas treatment using first gas which contains oxygen at a concentration over 0 vol% to not greater than 21 vol%; and
    subsequently, a step of supplying, to the reactor, second gas which contains water vapor at a concentration over 0 vol% to not greater than 42 vol% and oxygen at a concentration over 0 vol% to not greater than 21 vol%,

    wherein a temperature of a heat medium circulating in the reactor is in a range from not less than 200°C to below 400°C, and the temperature of the heat medium circulating in the reactor is fixed from an end of the gas treatment step until an end of the subsequent gas supply step, and
    wherein a water vapor content in the first gas and a water vapor content in the second gas are different.

2.  The catalyst regeneration method according to claim 1,
    wherein the gas supply step is conducted after a generation speed of $CO_2$ and CO discharged from the reactor has reached a maximum generation speed during the gas treatment step conducted under a condition defined above, when the generation speed of $CO_2$ and CO discharged from the reactor decreases to 95% or less of the maximum generation speed.

3.  The catalyst regeneration method according to claim 2,
    wherein a cycle comprising the gas treatment step and the subsequent gas supply step is repeated twice or more, and wherein a temperature of the heat medium during the gas treatment step and the gas supply step in a first cycle and a temperature of the heat medium during the gas treatment step and the gas supply step in a second cycle are different.

4.  The catalyst regeneration method according to claim 3,
    wherein the temperature of the heat medium during the gas treatment step and the gas supply step in the second cycle is higher than the temperature of the heat medium during the gas treatment step and the gas supply step in the first cycle.

5.  The catalyst regeneration method according to any one of claims 1 to 4,
    wherein the temperature of the heat medium circulating in the reactor is between not less than 200°C and not greater than 350°C.

6.  The catalyst regeneration method according to any one of claims 1 to 5,
    wherein the catalyst is a supported catalyst for butadiene production in which the catalyst for butadiene production is supported by a support.

## INTERNATIONAL SEARCH REPORT

| | International application No. |
|---|---|
| | PCT/JP2016/088406 |

A. CLASSIFICATION OF SUBJECT MATTER
*B01J38/20*(2006.01)i, *B01J23/887*(2006.01)i, *B01J23/94*(2006.01)i, *B01J38/02* (2006.01)i, *B01J38/12*(2006.01)i, *B01J38/16*(2006.01)i, *C07B61/00*(2006.01)n, *C07C5/48*(2006.01)n, *C07C11/167*(2006.01)n
According to International Patent Classification (IPC) or to both national classification and IPC

B. FIELDS SEARCHED

Minimum documentation searched (classification system followed by classification symbols)
B01J38/20, B01J23/887, B01J23/94, B01J38/02, B01J38/12, B01J38/16, C07B61/00, C07C5/48, C07C11/167

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus(JDreamIII), JST7580(JDreamIII), JSTChina(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | WO 2014/202501 A1 (BASF SE),<br>24 December 2014 (24.12.2014),<br>page 6, lines 11 to 28; page 11, line 33 to<br>page 15, line 7; claims 1, 5<br>& JP 2016-522229 A<br>claims 1, 5; paragraphs [0025], [0048] to<br>[0063]<br>& US 2016/0122264 A1<br>paragraphs [0049] to [0059], [0093] to [0110];<br>claims 16, 20<br>& EP 3010635 A1 | 1-2,5-6<br>3-4 |

☒ Further documents are listed in the continuation of Box C.          ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 February 2017 (08.02.17) | 21 February 2017 (21.02.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

**INTERNATIONAL SEARCH REPORT**

International application No.

PCT/JP2016/088406

C (Continuation).    DOCUMENTS CONSIDERED TO BE RELEVANT

| Category* | Citation of document, with indication, where appropriate, of the relevant passages | Relevant to claim No. |
|---|---|---|
| X<br>A | WO 2014/086768 A1 (BASF SE),<br>12 June 2014 (12.06.2014),<br>page 5, lines 25 to 27; page 15, line 1 to page 16, line 26; claims 1 to 3, 8<br>& JP 2016-502549 A<br>claims 1 to 3, 8; paragraphs [0024], [0075] to [0081]<br>& EP 2950928 A1 | 1-2,5-6<br>3-4 |
| X<br>A | WO 2015/004042 A2 (BASF SE),<br>15 January 2015 (15.01.2015),<br>page 5, lines 24 to 26; page 16, line 5 to page 17, line 38; claims 1, 4 to 5, 8<br>& JP 2016-526565 A<br>claims 1, 4 to 5, 8; paragraphs [0022], [0078] to [0085]<br>& US 2016/0152530 A1<br>paragraphs [0040], [0109] to [0116]<br>& EP 3019458 A2 | 1-2,5-6<br>3-4 |
| X<br>A | WO 2014/086815 A1 (BASF SE),<br>12 June 2014 (12.06.2014),<br>page 6, lines 12 to 15; page 16, line 10 to page 18, line 10; claims 1, 5 to 6, 11<br>& JP 2016-500372 A<br>claims 1, 5 to 6, 11; paragraphs [0026], [0078] to [0086]<br>& EP 2928849 A1 | 1-2,5-6<br>3-4 |
| A | WO 2015/007839 A1 (BASF SE),<br>22 January 2015 (22.01.2015),<br>page 6, line 33 to page 7, line 17; page 8, line 1 to page 9, line 2; claim 1<br>& JP 2016-525518 A<br>claim 1; paragraphs [0022] to [0024], [0027] to [0030]<br>& US 2016/0152532 A1<br>paragraphs [0046] to [0047], [0051] to [0064]; claim 8<br>& EP 3022168 A1 | 1-6 |
| A | JP 60-020987 A (JGC Corp.),<br>02 February 1985 (02.02.1985),<br>entire text<br>(Family: none) | 1-6 |
| A | JP 60-058928 A (Japan Synthetic Rubber Co., Ltd.),<br>05 April 1985 (05.04.1985),<br>claims; example 19<br>(Family: none) | 1-6 |

Form PCT/ISA/210 (continuation of second sheet) (January 2015)

**INTERNATIONAL SEARCH REPORT**

International application No.

PCT/JP2016/088406

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/157390 A1 (Asahi Kasei Chemicals Corp.), 02 October 2014 (02.10.2014), paragraphs [0033] to [0045]; examples; claims 1 to 6 & US 2016/0016864 A1 paragraphs [0038] to [0051]; examples; claims 1 to 20 & EP 2980053 A1 | 1-6 |
| A | CN 103071544 A (CHINA PETROLEUM AND CHEMICAL CORP.), 01 May 2013 (01.05.2013), entire text (Family: none) | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2222726 A
- JP 5192590 A
- JP 2005521021 A
- JP 2004522563 A
- JP 2003071299 A
- JP 58030340 A
- JP 2016502549 A
- JP 2016500372 A
- JP 2016526565 A
- JP 2015253054 A